# FASCICULE DE BREVET EUROPEEN

(11) **EP 4 340 719 B1**
(45) Date de publication et mention de la délivrance du brevet: **05.11.2025**
(21) Numéro de dépôt: 22724235.1
(22) Date de dépôt: 11.05.2022
(51) Int. Cl.: A61B 5/11, A61B 5/113, A61B 7/00, A61B 7/04

(54) **CAPTEUR DE VIBRATION ET DISPOSITIF POUR LA MESURE DE SIGNAUX VITAUX PERIODIQUES EMIS PAR LE CORPS HUMAIN OU ANIMAL**
VIBRATIONSSENSOR UND VORRICHTUNG ZUR MESSUNG VON PERIODISCHEN VITALSIGNALEN DES MENSCHLICHEN ODER TIERISCHEN KÖRPERS
VIBRATION SENSOR AND DEVICE FOR MEASURING PERIODIC VITAL SIGNALS EMITTED BY THE HUMAN OR ANIMAL BODY

(30) Priorité: 18.05.2021 FR 2105201
(43) Date de publication de la demande: 27.03.2024
(73) Titulaire: Wormsensing, 38040 Grenoble Cedex 9 (FR)
(72) Inventeur: LORNE, Thomas, 13510 Eguilles (FR); GUELFUCCI, Jude, 38100 Grenoble (FR); DEGIRMENCIOGLU, Ismail, 01130 Nantua (FR); BENAISSA, Lamine, 38000 Grenoble (FR)
(74) Mandataire: IP Trust
(86) Numéro de dépôt international: PCT/FR2022/050902
(87) Numéro de publication internationale: WO 2022/243624

(56) Documents cités:
- EP-A1- 3 144 074
- WO-A1-2016/026028
- CN-A- 106 500 826
- CN-A- 106 725 395
- US-A1- 2013 033 382
- DELOBELLE P ET AL: "True Young modulus of Pb(Zr,Ti)O3 films measured by nanoindentation", APPLIED PHYSICS LETTERS, AMERICAN INSTITUTE OF PHYSICS, 2 HUNTINGTON QUADRANGLE, MELVILLE, NY 11747, vol. 85, no. 22, 1 January 2004 (2004-01-01), pages 5185 - 5187, XP012063583, ISSN: 0003-6951, DOI: 10.1063/1.1827331
- TSUCHIYA KAZUYOSHI ET AL: "Fabrication of Smart Material PZT Thin Films by RF Magnetron Sputtering Method in Micro Actuators", JSME INTERNATIONAL JOURNAL. SERIES A, SOLID MECHANICS AND MATERIAL ENGINEERING, 1 January 2006 (2006-01-01), Tokyo, pages 201 - 208, XP055871211, Retrieved from the Internet <URL:https://www.jstage.jst.go.jp/article/jsmea/49/2/49_2_201/_pdf/-char/en> [retrieved on 20211209], DOI: 10.1299/jsmea.49.201

## Description

### DOMAINE DE L'INVENTION

La présente invention concerne le domaine de la collecte de signaux vitaux périodiques émis par le corps humain/animal, signaux incluant notamment les pulsations cardiaques et le rythme respiratoire. Elle concerne en particulier un capteur de vibration et un dispositif équipé dudit capteur, permettant la mesure desdits signaux vitaux périodiques, en étant appliqué contre le corps, en contact avec la peau ou avec une couche intermédiaire (vêtements, fourrures, cuir, etc), et ce dans des environnements calmes ou bruyants.

### ARRIERE PLAN TECHNOLOGIQUE DE L'INVENTION

La phonocardiographie est une méthode acoustique non invasive de mesure du rythme cardiaque. A partir des phonocardiogrammes (PCG) obtenus avec cette méthode, il est possible, après analyse, de déduire différents indicateurs renseignant sur le fonctionnement général du cœur et/ou l'état du sujet (état de santé, stress, émotions, fatigue, etc...). Par exemple, la variabilité de la fréquence cardiaque (HRV) est un indicateur particulièrement pertinent et fréquemment utilisé pour déterminer les réponses physiologiques liées aux émotions, au stress, à la fatigue ou à l'endormissement.

Bien qu'éprouvée et reconnue par la communauté scientifique, la phonocardiographie présente néanmoins certaines contraintes qui limitent aujourd'hui la généralisation de son usage. En effet, les dispositifs actuels nécessitent, pour la plupart, un contact intime avec le sujet (directement sur la peau), une connaissance du positionnement adéquat du dispositif de mesure et de la pression à exercer sur la peau, et un environnement sonore calme. C'est notamment le cas du dispositif portable décrit dans le document KR101957110, qui comprend un capteur en céramique piézoélectrique, destiné à être directement en contact avec la peau de l'utilisateur, et un anneau adhésif permettant de maintenir le contact avec la peau. Le document CN106500826 décrit un capteur de micro-mouvement pour l'acquisition d'un signal physiologique, comprenant en particulier une structure mécanique d'amplification de la vibration à mesurer.

### OBJET DE L'INVENTION

La présente invention vise à remédier à tout ou partie des inconvénients précités. Elle concerne en particulier un dispositif compact, apte à capter et analyser lesdits signaux vitaux périodiques, en étant appliqué contre le corps d'un individu, en contact avec la peau ou avec une couche intermédiaire (vêtements, fourrures, cuir, etc), et ce, dans des environnements calmes ou bruyants, que le sujet soit immobile ou en mouvement.

### BREVE DESCRIPTION DE L'INVENTION

L'invention concerne un capteur de vibration selon la revendication 1.

Selon d'autres caractéristiques avantageuses et non limitatives de l'invention, prises seules ou selon toute combinaison techniquement réalisable :
- le capteur de vibration comprend une couche d'adaptation d'impédance, présentant une impédance acoustique comprise entre 5.10⁵ Pa*s/m et 3.10⁶ Pa*s/m, et disposée sur une face de la couche support opposée à celle en contact avec la couche de connexion électrique ;
- le matériau piézoélectrique de la couche active est choisi parmi les céramiques sous forme monocristalline, poly-cristalline ou composite ;
- les électrodes de contact présentent une épaisseur cumulée inférieure à deux fois l'épaisseur de la couche active ;
- la couche d'adaptation d'impédance présente une épaisseur supérieure ou égale à 10 microns ;
- la couche de connexion électrique est formée par un interposeur ou par un film conductif anisotrope ;
- la couche support inclut une membrane disposée sur une face du circuit imprimé opposée à celle en contact avec la couche de connexion électrique ;
- l'empilement de couches et la couche support présentent respectivement une première superficie et une deuxième superficie, dans le plan principal, la première superficie étant inférieure ou égale à 30% de la deuxième superficie ;
- la couche support comprend une structure de rigidification, solidaire d'une zone périphérique de ladite couche support ;
- le circuit imprimé comprend un élément de connexion filaire, pour raccorder le capteur de vibration à un terminal électronique ;
- la structure de rigidification supporte deux prises de contact électrique, chacune reliée à une borne électrique, pour raccorder le capteur de vibration à un terminal électronique ;
- le capteur de vibration comprend un joint périphérique ;
- le capteur de vibration comprend une couche de protection disposée au-dessus et à distance de l'empilement de couches, ladite couche de protection étant solidaire de la couche support.

L'invention concerne également un dispositif, non intrusif, pour la mesure d'au moins un signal vital périodique d'un individu, comprenant :
- au moins un capteur de vibration tel que ci-dessus, pour mesurer un signal brut représentatif du signal vital périodique, et
- un terminal électronique raccordé audit capteur de vibration, pour analyser et interpréter le signal brut et extraire le signal vital périodique ou un paramètre de sortie représentatif dudit signal vital périodique. Le terminal électronique comprend avantageusement :
- un étage analogique de conditionnement du signal brut mesuré par le capteur de vibration,
- un étage de conversion analogique à digital du signal issu de l'étage de conditionnement,
- un étage de traitement du signal digital, pour la mise en forme du signal digital et le calcul d'un paramètre de sortie représentatif dudit signal vital,
- et potentiellement, un étage de communication avec un système externe.

### BREVE DESCRIPTION DES FIGURES

D'autres caractéristiques et avantages de l'invention ressortiront de la description détaillée de l'invention qui va suivre en référence aux figures annexées :
- Les figures 1a et 1b présentent un capteur de vibration conforme à l'invention, respectivement en coupe schématique et en perspective ;
- Les figures 2a, 2b et 2c présentent un capteur de vibration conforme à l'invention, respectivement en coupe schématique et en perspective ;
- Les figures 3a et 3b présentent un capteur de vibration conforme à l'invention, respectivement en coupe schématique et en perspective ;
- La figure 4 présente différentes formes, en vue de dessus, d'un capteur de vibration conforme à l'invention ;
- La figure 5 présente différentes configurations de dispositifs pour la mesure d'un signal vital périodique, conformes à l'invention ;
- La figure 6a présente un spectrogramme A capté par un capteur de vibration conforme à l'invention et un spectrogramme Ref capté par un microphone classique ; la figure 6b présente un spectrogramme B issu du spectrogramme A après application d'un filtre en fréquences ; et un signal vital C,D sous forme d'onde capté et traité par le dispositif conforme à l'invention.

Les mêmes références sur les figures pourront être utilisées pour des éléments de même type. Certaines figures comportent des représentations schématiques qui, dans un objectif de lisibilité, ne sont pas à l'échelle : en particulier, les épaisseurs des couches selon l'axe z ne sont pas à l'échelle par rapport aux dimensions latérales selon les axes x et y ; et les épaisseurs relatives des couches entre elles ne sont pas nécessairement respectées.

Les différentes possibilités (variantes et modes de réalisation illustrés et/ou détaillés dans la description à suivre) doivent être comprises comme n'étant pas exclusives les unes des autres et peuvent se combiner entre elles.

### DESCRIPTION DETAILLEE DE L'INVENTION

L'invention concerne un capteur de vibration 100 pour la mesure d'au moins un signal vital périodique, régulier ou irrégulier, d'un individu. L'individu est considéré ici au sens large et peut correspondre à un être humain ou un animal. Le signal vital périodique peut notamment être le rythme cardiaque ou le rythme respiratoire.

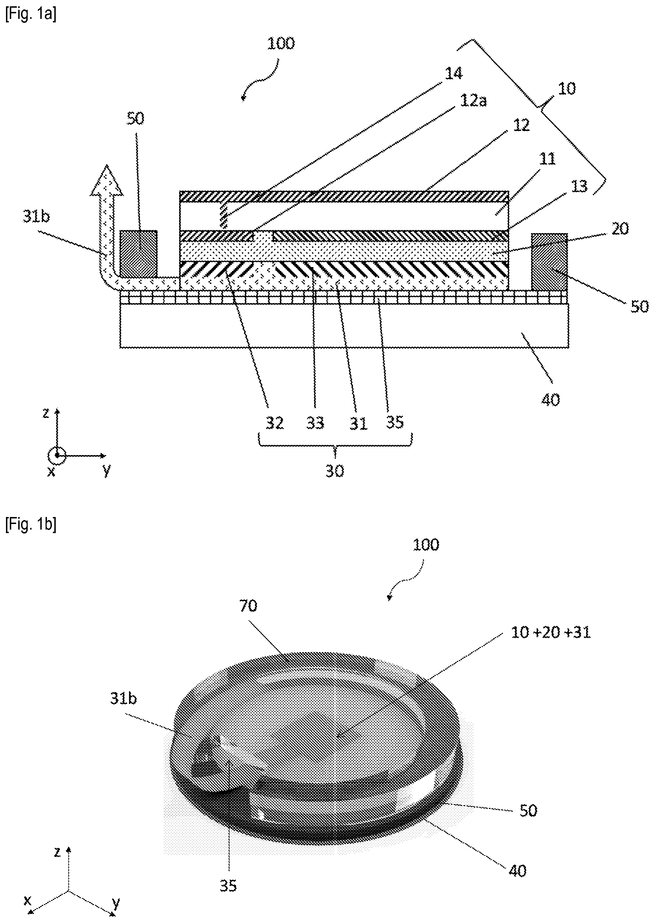

Différentes configurations de capteurs de vibration 100 conformes à la présente invention sont illustrés sur les figures 1a, 1b, 2a, 2b, 3a et 3b.

Le capteur de vibration 100 comprend un empilement de couches 10 s'étendant parallèlement à un plan principal (x,y), c'est-à-dire que les faces principales de cet empilement 10 sont sensiblement parallèles au plan principal (x,y) et que l'épaisseur de l'empilement 10 se mesure selon un axe z normal audit plan principal. La dénomination de couche, dans la présente invention, sous-entend que l'épaisseur de la couche (ou de l'empilement de couches) est, en général, significativement inférieure aux dimensions latérales (dans le plan principal) de ladite couche.

L'empilement de couches 10 inclut une couche active 11 en matériau piézoélectrique qui présente une épaisseur inférieure ou égale à 20 microns et un module d'Young supérieur ou égal à 60 GPa. Ces caractéristiques physiques confèrent à la couche active 11 un haut niveau de sensibilité et au capteur 100 un fort ratio signal sur bruit, pour la détection d'ondes acoustiques dans les fréquences relatives aux signaux vitaux périodiques visés. La faible épaisseur de la couche active 11 favorise également la compacité du capteur 100.

Comme cela est connu en soi, la couche active 11 en matériau piézoélectrique va se polariser (et donc générer une circulation de charges conduisant à un signal électrique mesurable) si elle subit une déformation, en particulier ici, déformation provoquée par la pulsation du signal vital périodique.

Avantageusement, l'épaisseur de la couche active 11 est inférieure ou égale à 10 microns, voire même inférieure ou égale à 5 microns, pour améliorer encore la sensibilité de détection des ondes acoustiques. On s'assurera de conserver une épaisseur de couche active 11 suffisante pour générer des tensions de polarisation typiquement supérieures à 500 microvolts lors d'une déformation.

Les dimensions latérales (dans le plan principal (x,y)) de la couche active 11 peuvent par exemple être choisies entre 500 microns et 50 mm, des faibles dimensions étant bien sûr privilégiées pour des questions de compacité du capteur de vibration 100.

Le matériau de la couche active 11 est préférentiellement choisi parmi les céramiques piézoélectriques, sous une forme monocristalline, poly-cristalline ou composite (correspondant à une dispersion de poudre céramique piézoélectrique dans une matrice, polymère généralement). A titre d'exemple, on peut citer les céramiques suivantes : niobate de lithium (LiNbO₃), tantalate de lithium (LiTaO₃), niobate de potassium (KNbO₃), (BaTiO₃), quartz (SiO₂), niobate de magnésium-plomb titanate de plomb (PMN-PT), titano-zirconate de plomb (PZT), matériaux à base de potassium sodium niobium lithium antimoine (KNN-LS) ou modifiés par du titanate de calcium (KNN-LS-CT), matériaux à base de potassium sodium lithium niobium tantale antimoine (KNLNTS), titanate de bismuth sodium (BNKLBT), etc.

L'empilement de couches 10 inclut également deux électrodes de contact 12,13, disposées sur une des faces de la couche active 11 ou sur les deux faces (à savoir de part et d'autre de la couche active 11), pour permettre la libre circulation des charges, mises en mouvement par la polarisation (représentative du signal vital périodique) de ladite couche 11.

Préférentiellement, les électrodes de contact 12,13 présentent une épaisseur cumulée inférieure à deux fois l'épaisseur de la couche active 11, voire inférieure à l'épaisseur de la couche active 11 ; chaque électrode 12,13 présente donc avantageusement une épaisseur inférieure à 10 microns, voire inférieure à 5 microns.

Les électrodes de contact 12,13 pourront être formées à partir de matériaux métalliques purs (par exemple, Ag, Au, Pd, Pt, Cu, Ni, W ou Ti), d'alliages conducteurs ou de matériaux conducteurs 2D (par exemple, le graphène). Une barrière de diffusion (par exemple, en TiN, WN ou TaN) et une couche d'accroche (par exemple, en Cr ou Ti) pourront être prévues entre le matériau conducteur de chaque électrode 12,13 et la couche active 11.

Avantageusement, l'empilement de couches 10 est constitué de la couche active 11 et des deux électrodes de contact 12,13 uniquement.

Le capteur de vibration 100 comprend également une couche support 30, flexible, s'étendant parallèlement au plan principal (x,y) et incluant un circuit imprimé 31 comportant deux bornes électriques 32,33. Une couche de connexion électrique 20 (qui fait également partie du capteur de vibration 100) est disposée entre l'empilement de couches 10 et la couche support 30, pour connecter chaque électrode de contact 12,13 à une borne électrique 32,33.

La couche de connexion électrique 20 est avantageusement formée par un interposeur ou par un film conductif anisotrope (ACF). Dans tous les cas, l'objectif est que les deux électrodes de contact 12,13 de l'empilement de couches 10 puissent être atteintes au niveau d'une seule et même face de l'empilement 10 ; cette face (dite face inférieure) étant ensuite associée à la couche de connexion 20. Dans le cas où les électrodes de contact 12,13 sont respectivement disposées sur la face inférieure et l'autre face (dite face supérieure) de la couche active 11, il est avantageux de prévoir un via conducteur 14 traversant ladite couche active 11 et reliant électriquement l'électrode 12, disposée sur la face supérieure, à un plot 12a disposé sur la face inférieure et isolé électriquement de l'autre électrode 13 également disposée sur la face inférieure.

Un interposeur peut être composé de résine thermoplastique (isolant) et d'un matériau conducteur électrique (par exemple, Nikel) permettant le raccordement entre chaque électrode de contact 12,13 et une borne électrique 32,33.

Un film conductif anisotrope est classiquement composé de billes conductrices dispersées dans une matrice polymère isolante ; lorsqu'une pression ou une thermocompression est appliquée à l'ensemble empilement de couches 10 / ACF 20 / couche support 30, une conduction électrique verticale s'établit entre électrodes 12a,13 et bornes 32,33 (habituellement en surépaisseur) via les billes conductrices, alors que les zones intercalaires restent isolantes.

Il existe également des adhésifs conductifs anisotropes (ACA) qui pourraient être utilisés pour former la couche de connexion électrique 20. Ces adhésifs sont basés sur le même principe que le film conductif anisotrope (ACF) précité, à l'exception du fait que la matrice polymère est remplacée par un précurseur liquide capable d'être thermiquement activé pour former le polymère final (par polymérisation) ; le résultat final reste similaire à l'ACF (billes conductrices dispersées dans une matrice isolante), mais compte tenu du fait que l'application se fait en phase liquide, il est possible de réduire drastiquement l'épaisseur de la couche de connexion électrique 20.

Une solution plus basique peut également être envisagée : à savoir la mise en œuvre d'une pate conductrice pour connecter chaque électrode et plot de la face inférieure, à une borne 32,33 associée, et d'un matériau de remplissage isolant pour isoler électriquement les électrodes 12a,13 entre elles et les bornes 32,33 entre elles.

La couche de connexion électrique 20 est uniquement en contact avec l'une des faces principales de l'empilement de couches 10 ; les bords et l'autre face principale de l'empilement de couches 10 sont totalement libres, sans contact mécanique avec la couche de connexion 20.

La couche de connexion électrique 20 est donc au moins en partie composée d'un matériau conducteur électrique et procure une connexion verticale directe entre électrodes et bornes, à l'inverse d'une connexion par exemple par câbles ou fils éventuellement enrobés dans un isolant. L'absence de câbles améliore la sensibilité du capteur de vibration 100, en évitant l'introduction de raideur supplémentaire dans la structure, liée aux câbles et aux soudures associées.

Préférentiellement, la couche de connexion électrique 20 est donc en contact direct et homogène contre l'intégralité d'une face principale de l'empilement de couches 10. Du côté de son autre face, la couche 20 est avantageusement en contact direct et homogène contre une face de la couche support 30.

La couche de connexion électrique 20 présente typiquement une épaisseur inférieure à 50 microns, en particulier une épaisseur comprise entre 1 micron et 10 microns.

La couche support 30 est une couche autoportée, qui présente une épaisseur inférieure ou égale à 500 microns. Cela lui confère la flexibilité requise.

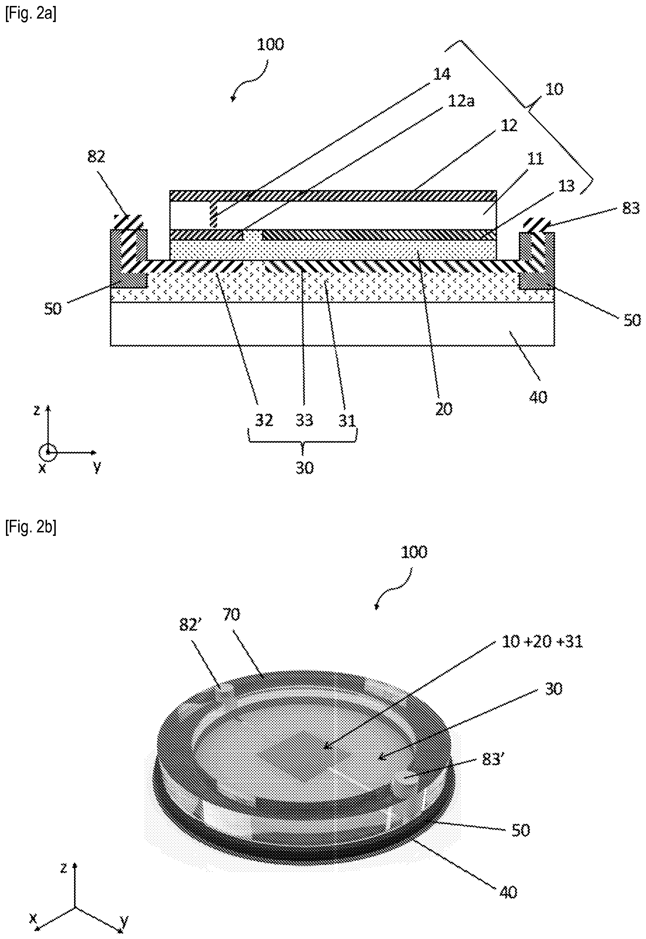

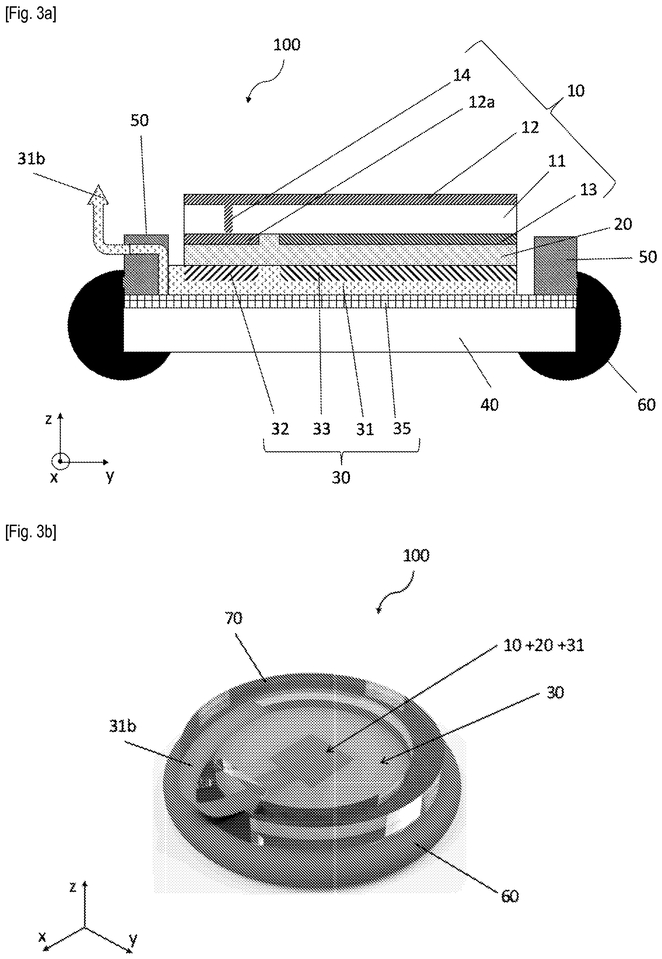

Selon une variante, la couche support 30 est essentiellement composée par le matériau formant le circuit imprimé 31 (figure 2a) : par exemple, un composite de résine époxyde renforcé de fibres de verre. Selon une autre variante, la couche support 30 comprend également une membrane 35, le circuit imprimé 31 étant alors situé entre la membrane 35 et la couche de connexion électrique 20 (figures 1a et 3a). Le matériau de la membrane 35, et son épaisseur, peuvent ainsi être choisis et ajustés de manière à conférer la flexibilité visée à la couche support 30. La membrane 35 peut par exemple être formée en métal, en chlorure de polyvinyle (PVC), ou en époxy et fibres de verres. A titre d'exemple, la membrane 35 (lorsqu'elle est présente) peut avoir une épaisseur comprise entre 50 et 300 microns, et le circuit imprimé 31 peut avoir une épaisseur comprise entre 30 et 200 microns.

La couche support 30 présente une raideur comprise entre 1150000 N/m et 6900000 N/m. Le caractère flexible de la couche support 30, lié à son épaisseur et à sa raideur, permet de transmettre efficacement une déformation à la couche active 11, à chaque pulsation du signal vital.

De manière avantageuse, l'empilement de couches 10 et la couche support 30 présentent respectivement une première superficie et une deuxième superficie, dans le plan principal (x,y), la première superficie étant inférieure ou égale à 30% de la deuxième superficie. L'empilement de couches 10 peut être disposé en partie centrale de la couche support 30, notamment pour une facilité d'assemblage, ou en périphérie pour interférer le moins possible avec la déformation de ladite couche support 30, générée par la pulsation périodique du signal vital que l'on cherche à mesurer ; l'objectif global est d'optimiser la déformation subie par l'empilement de couches 10, cela en fonction de la géométrie du capteur de vibration 100.

Selon un premier mode de mise en œuvre du capteur conforme à l'invention, la couche support 30 est destinée à être en contact avec l'individu (contre sa peau ou contre son vêtement ou fourrure) : la couche support 30 va alors se déformer du fait de la pulsation périodique du signal vital, et transmettre cette déformation à la couche active 11 de l'empilement 10.

Selon un deuxième mode de mise en œuvre, le capteur de vibration 100 comprend en outre une couche d'adaptation d'impédance 40, qui présente une impédance acoustique idéalement comprise entre 5.10⁵ Pa*s/m et 3.10⁶ Pa*s/m. Cette impédance acoustique est sciemment choisie proche de l'impédance acoustique des muscles et de la graisse (impédance comprise entre 1,3.10⁶ et 1,5.10⁶ Pa*s/m), de manière à favoriser la transmission des pulsations du signal vital à la couche support 30. Par exemple, la couche d'adaptation d'impédance 40 peut être formée en silicone (impédance acoustique 1,6.10⁶ Pa*s/m) ou en bioplastique, par exemple de marque Ecoflex^{®} (impédance acoustique 1,053.10⁶ Pa*s/m).

La couche d'adaptation d'impédance 40 est disposée contre la couche support 30, sur une face de ladite couche support 30 opposée à celle en contact avec la couche de connexion électrique 20. La couche d'adaptation d'impédance 40 présente typiquement une épaisseur supérieure ou égale à 10 microns, par exemple comprise entre 50 microns et 5 mm. Lorsque la couche support 30 comprend une membrane 35, cette dernière est en contact avec la couche d'adaptation d'impédance 40.

La couche d'adaptation d'impédance 40 est destinée à être en contact avec l'individu (contre sa peau ou contre son vêtement ou fourrure). En plus de transmettre efficacement les pulsations du fait de son appariement d'impédance avec les tissus corporels, cette couche 40 favorise également le maintien du capteur 100 contre l'individu car son matériau souple et déformable a tendance à « adhérer » à la surface de contact, par frottement d'adhérence. La présence de la couche d'adaptation d'impédance 40, dans le deuxième mode de mise en œuvre du capteur 100, est donc particulièrement favorable lorsque l'environnement de mesure est bruyant autour de l'individu dont il faut capter le signal vital, et/ou lorsque l'individu est en mouvement.

Dans l'un ou l'autre des modes de mise en œuvre décrits, il peut être avantageux que le capteur de vibration 100 comprenne un joint périphérique 60 entourant au moins la couche d'adaptation d'impédance 40 (lorsqu'elle est présente), comme illustré sur les figures 3a et 3b, ou entourant tout ou partie de la couche support 30 (en l'absence de couche d'adaptation d'impédance 40). Ce joint 60 permet d'accommoder la topologie locale lorsque le capteur 100 est placé au contact de l'individu.

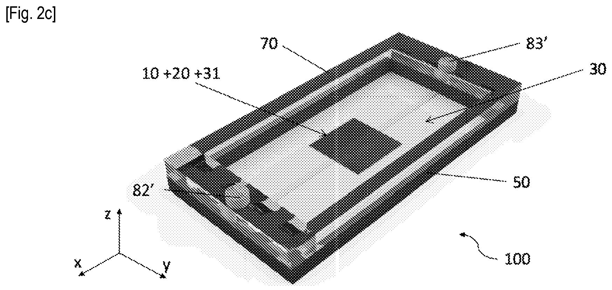

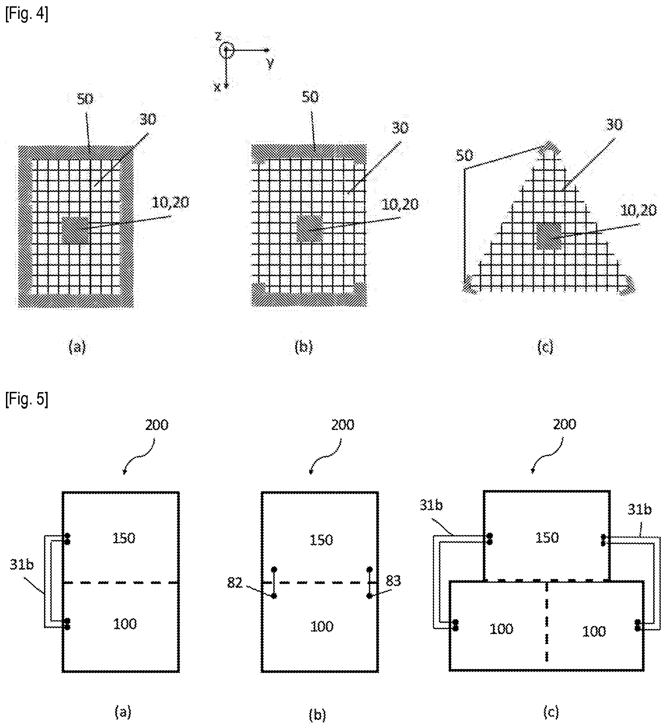

La couche support 30 du capteur de vibration 100 peut également comprendre une structure de rigidification 50, solidaire d'une zone périphérique de la couche support 30. La structure de rigidification 50 a pour fonction d'immobiliser la périphérie de la couche support 30 et de la couche d'adaptation d'impédance 40 (si elle est présente), et ainsi d'accentuer leur déformation générée par la pulsation périodique du signal vital que l'on cherche à mesurer. La structure de rigidification 50 peut prendre diverses formes telles que par exemple :
- un cadre continu (figure 4(a)), avantageusement un anneau (comme illustré sur la figure 1b et la figure 2b), mais éventuellement un rectangle (figure 2c), un triangle ou autre polygone ; ou
- un cadre discontinu, composé de deux zones rigides (figure 4(b)), de trois zones rigides (figure 4(c)), voire plus.

La structure de rigidification est avantageusement formée dans un matériau présentant une dureté supérieure à 30 Shore D, tel que le PET (polytéréphtalate d'éthylène), PMMA (polyméthacrylate de méthyle), PU (polyuréthane), PVC (polychlorure de vinyle), PP (polypropylène), etc.

En outre, compte tenu de l'épaisseur totale réduite de l'ensemble comprenant l'empilement de couches 10, la couche de connexion 20, la couche support 30 et potentiellement la couche d'adaptation d'impédance 40, il peut être judicieux de prévoir un système facilitant la manipulation du capteur 100 et favorisant sa robustesse : la structure de rigidification 50 participe à un tel système.

Toujours dans le but d'améliorer la robustesse du capteur de vibration 100 et de protéger notamment la couche active 11, le capteur 100 est préférentiellement muni d'une couche de protection 70 disposée au-dessus et à distance de l'empilement de couches 10. La couche de protection 70 peut avantageusement être solidaire de la structure de rigidification 50. Comme cela est illustré sur les figures 1b, 2b, 2c et 3b, cette couche de protection 70 peut notamment consister en une coque en plastique, par exemple de 500 microns d'épaisseur. Parce qu'elle est située à distance (selon l'axe z sur les figures) de l'empilement de couches 10 (sans contact avec l'empilement, donc), elle ne perturbe pas la déformation de celui-ci en liaison avec la couche support 30.

Notons que les figures 1b, 2b et 3b illustrent des capteurs de vibration 100 de forme générale circulaire, dans le plan principal (x,y), comprenant un empilement de couches 10 de forme carrée. Toute autre forme, tant pour l'empilement de couches 10 que pour la couche support 30 (et pour les autres couches de l'ensemble formant le capteur 100) est bien sûr envisageable.

Le capteur de vibration 100 selon l'invention est défini pour faire partie d'un dispositif 200 non intrusif, pour la mesure d'au moins un signal vital périodique d'un individu. Un tel dispositif 200, également objet de la présente invention, comprend au moins un capteur de vibration 100 tel que décrit précédemment, pour mesurer un signal brut (lié au signal vital périodique caractéristique de l'individu), et un terminal électronique 150 raccordé audit capteur de vibration 100, pour analyser et interpréter le signal brut puis extraire le signal vital périodique ou des informations relatives à ce signal vital. Le dispositif 200 peut comporter un capteur de vibration 100 (figure 5 (a), (b)) ou une pluralité (deux, voire plus) de capteurs 100 raccordés au terminal électronique 150 (figure 5 (c)). Lorsqu'il y a plusieurs capteurs 100, il est possible de mesurer un même signal ou des signaux vitaux différents (rythme cardiaque et respiration), d'un même individu ou de plusieurs individus (comme par exemple, une femme enceinte et son bébé).

Comme évoqué précédemment, la couche support 30 ou, lorsqu'elle est présente, la couche d'adaptation d'impédance 40 du capteur de vibration 100, est destinée à être disposée contre l'individu, soit directement contre la peau, soit contre un vêtement (être humain), une fourrure (animal), ou autre. La sensibilité du capteur de vibration 100 permet de capter un signal brut, quelle que soit la configuration (contact peau ou couche intermédiaire de type vêtement).

Le capteur de vibration 100 selon l'invention présente en outre l'intérêt d'atténuer fortement les fréquences situées en-dehors de la gamme de fréquences d'intérêt (gamme de fréquences typiquement entre 0,2 Hz et 500 Hz pour les rythmes cardiaque et respiratoire, voire fréquences inférieures ou égales à 70 Hz): il a en particulier été observé que la parole et d'autres sons d'environnement ne viennent pas polluer le signal mesuré, tout particulièrement dans le cas du deuxième mode de mise en œuvre impliquant la couche d'adaptation d'impédance 40. L'environnement sonore de l'individu au moment de la prise de mesure n'a donc pas besoin d'être calme et silencieux ; l'individu n'a pas non plus besoin de demeurer immobile. Cela étend grandement les possibilités de suivi des signaux vitaux et ce, dans des conditions moins contraignantes qu'avec les dispositifs de l'état de la technique.

On choisira, de préférence, de placer le capteur de vibration 100 (à savoir, la couche support 30 ou éventuellement la couche d'adaptation d'impédance 40) contre une zone du corps au niveau de laquelle la pulsation vitale (rythme respiratoire, rythme cardiaque) que l'on souhaite mesurer, est palpable au toucher.

Pour connecter le capteur de vibration 100 et le terminal électronique 150, le circuit imprimé 31 du capteur 100 peut comprendre un élément de connexion filaire 31b, par exemple un cordon sous forme de nappe, comme illustré sur les figures 1a,1b et 3a,3b. L'embout de l'élément de connexion filaire 31b comporte des prises de contact électrique, reliées aux bornes électriques 32,33 du circuit imprimé 31, qui peuvent être connectées au terminal électronique 150. Le terminal électronique 150 peut, dans ce cas, être situé à distance du capteur 100, notamment sur un module de fixation à l'individu (par exemple une poche, une ceinture, un bracelet... ou autre). Toujours à distance du capteur 100, le terminal électronique peut être connecté ou intégré à un système externe plus complexe, tel qu'un moniteur, fixe ou éventuellement transportable.

Alternativement, la structure de rigidification 50 du capteur de vibration 100 peut être le support de deux prises de contact électrique 82,83, chacune reliée à une borne électrique 32,33 du circuit imprimé 31, comme cela est visible sur la figure 2a. Dans un tel cas, le terminal électronique 150 peut être directement superposé sur la structure de rigidification 50, et connecté électriquement au capteur 100 via les deux prises de contact électrique 82,83. Selon une variante, la couche de protection 70 peut procurer des prises de contact intermédiaires 82',83', relayant les prises de contact électrique 82,83 et destinées à être connectées au terminal électronique 150 (figure 2b, figure 2c) ; ce dernier peut alors être directement disposé sur la couche de protection 70. Dans ces configurations, où le terminal 150 est superposé au capteur 100, le dispositif 200 peut prendre une forme particulièrement compacte et former un dispositif portable et potentiellement autonome.

Le terminal 150 comprend différents étages électroniques lui permettant d'analyser et d'interpréter le signal brut mesuré par le capteur de vibration 100. Un étage analogique de conditionnement du signal brut mesuré par le capteur de vibration 100 va tout d'abord amplifier et filtrer le signal électrique reçu du capteur 100. Cet étage est typiquement composé d'un premier bloc de type amplification de charge dont le rapport des résistances fixe le gain d'amplification du signal électrique reçu du capteur 100, et d'un deuxième bloc de type filtre Sallen & Key permettant de filtrer les fréquences au-delà du spectre acoustique des signaux vitaux visés. Le terminal électronique 150 comprend ensuite un étage de conversion analogique à digital, du signal issu de l'étage de conditionnement. Puis, un étage de traitement du signal digital, composé d'un microcontrôleur, réalise la mise en forme du signal par le calcul d'une fonction enveloppe de type énergie de Shanon. Enfin, à partir du signal mis en forme, le paramètre de sortie d'intérêt, représentatif dudit signal vital, peut être calculé.

Les données collectées, relatives au signal vital ou au paramètre de sortie d'intérêt, peuvent être stockées pour une analyse ultérieure, ou être interprétées en temps réel et déclencher la réponse d'un système secondaire compris dans le dispositif 200 ou externe. La réponse pourrait être un retour d'information (visuelle, acoustique, mécanique, etc) et/ou le déclenchement d'une ou plusieurs actions, par exemple :
- mécanique(s) : ouverture / fermeture d'un système,
- électrique(s) : allumage / extinction / variation d'un système, hydraulique, pneumatique, thermique, etc.

Pour autoriser la transmission du paramètre de sortie d'intérêt vers un potentiel système externe, le terminal électronique 150 peut comprendre un étage de communication. Des protocoles connus de connexion (CAN, UART, USB) ou de transmission de données sans fil, (Wi-Fi, bluetooth, etc) peuvent par exemple être utilisés.

Dans le cas d'un dispositif 200 portable, on pourra prévoir une batterie, préférentiellement rechargeable, pour alimenter en énergie les différents étages précités du terminal électronique 150.

Le capteur de vibration 100 et le dispositif 200 non intrusif de mesure d'un signal vital périodique selon la présente invention peuvent adresser nombre de champs applicatifs, dans les domaines de la médecine, la santé, le transport, l'industrie, le sport ou les loisirs.

Comme évoqué précédemment, le dispositif 200 peut se décliner sous différentes configurations :
- un dispositif portable et autonome, sous une forme compacte (capteur 100 et terminal 150 superposés) ou sous une forme dissociée (capteur 100 connecté au terminal 150 par un élément de connexion filaire 31b) ;
- un dispositif portable, sous une forme compacte ou sous une forme dissociée, dans lequel le terminal 150 est configuré pour être raccordé en filaire à un système externe plus complexe (notamment un moniteur), par exemple dans un véhicule de secours ou dans une salle d'examen médical ;
- un dispositif fixe, sous une forme compacte ou sous une forme dissociée, dans lequel le terminal 150 est raccordé en filaire ou intégré à un système externe fixe et plus complexe.

### Exemple de réalisation :

Un exemple de fabrication du capteur de vibration 100 et du dispositif 200 va maintenant être décrit. Bien sûr, cet exemple n'est pas limitatif car il existe d'autres procédés pour empiler et assembler différents types de couches, susceptibles d'être mis en œuvre pour réaliser les capteur 100 et dispositif 200, objets de l'invention.

Pour fabriquer l'empilement de couches 10 du capteur de vibration 100, il est notamment possible d'utiliser un procédé de transfert voisin de celui décrit par T.Dufay et al dans la publication « Flexible PZT thin film transferred on polymer substrate » (Surface and Coatings Technology, Elsevier, 2018, 343, pp.148-152).

Une solution de précurseur du PZT est déposée par centrifugation (« spin-coating ») sur un substrat sacrificiel (par exemple, en aluminium), pour former une couche visqueuse. Une ouverture est réalisée à travers ladite couche afin de permettre le passage d'une voie électrique. Puis, un traitement thermique à 650°C est appliqué pour cristalliser le PZT et former une couche active 11 en matériau piézoélectrique d'épaisseur 5 microns.

Une électrode de contact 12 en platine, d'épaisseur 400 nm, est déposée par une technique de dépôt chimique en phase vapeur (par exemple PECVD) sur la face supérieure (libre) de la couche active 11 en PZT, puis recouverte par une couche adhésive en polyuréthane. Une ouverture est également réalisée à travers l'empilement électrode / couche adhésive pour le passage de la voie électrique. Une couche temporaire en polymère (par exemple du PET), d'épaisseur 200 microns, est attachée à la couche adhésive en polyuréthane par thermocompression, pour faciliter la manipulation de la couche active 11. La couche temporaire est ouverte pour permettre le passage de la voie électrique, et remplie par de la colle conductrice, qui formera le via conducteur 14, en contact électrique avec l'électrode de contact 12. Le substrat sacrificiel est ensuite gravé chimiquement jusqu'à mettre à nu la face inférieure de la couche active 11 en PZT. L'autre électrode de contact 13 et le plot 12a, en contact électrique avec le via 14, sont formés par dépôt d'aluminium (environ 400 nm) sur ladite face inférieure du PZT.

Ce procédé de fabrication peut permettre l'élaboration d'un film de PZT présentant de grandes dimensions latérales, que l'on vient ensuite découper pour définir la couche active 11 aux dimensions latérales souhaitées pour son intégration dans le capteur de vibration 100 selon l'invention. Dans l'exemple décrit, la couche active 11 présente des dimensions latérales (selon le plan principal (x,y)) de 5 mm par 15 mm.

On choisit ensuite un circuit imprimé (PCB) 31 présentant une épaisseur de 100 microns, des dimensions latérales sensiblement identiques à celles de la couche active 11 et comportant deux bornes électriques 32,33. Un film conductif anisotrope (ACF) 20 est laminé sur le circuit imprimé 31. A l'aide d'une machine de manipulation (de type « Pick and Place »), la couche active 11 est positionnée en vis-à-vis de la couche de connexion 20, de sorte que chaque électrode 12a,13 (sur la face inférieure de la couche active 11) se trouve à l'aplomb d'une borne électrique 32,33 du circuit imprimé 31 ; puis un assemblage par thermocompression est opéré.

La couche temporaire en polymère peut alors être retirée.

Le circuit imprimé 31 est ensuite collé sur une membrane 35 en PVC, d'épaisseur 300 microns et de dimensions latérales 50 mm, pour finaliser la formation de la couche support 30.

Une couche d'adaptation d'impédance 40 en silicone, d'épaisseur 3 mm, peut être assemblée par laminage, sérigraphie ou moulage contre la membrane 35.

Une structure de rigidification 50 en polypropylène et un joint périphérique 60 en silicone sont fixés sur le pourtour de la membrane 35 par emboitement. Un capot en polypropylène, formant la couche de protection 70 au-dessus et à distance de la couche active 11, est coulé, injecté ou laminé sur la structure de rigidification 50.

Dans cet exemple, le circuit imprimé 31 comprend un élément filaire 31b (nappe) qui permet de relier les bornes électriques 32,33 du circuit imprimé 31 au terminal électronique 150, via des prises de contact électrique. Le terminal 150 comprend les étages électroniques énoncés dans la description générale.

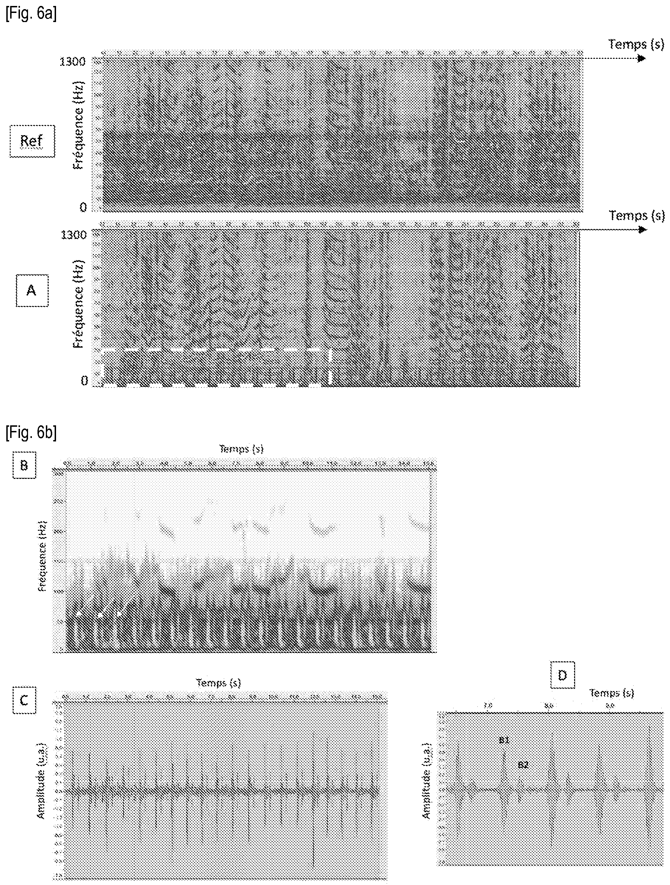

Avec le dispositif 200 ainsi formé, un exemple d'application à la mesure du rythme cardiaque d'un être humain est illustré sur les figures 6a et 6b.

Le cycle cardiaque comprend deux phases : la première est une phase de contraction (systole) et la seconde est une phase de relaxation (diastole). Au cours de la systole, le sang est éjecté des cavités du cœur, et pendant la diastole, les cavités se remplissent de sang. La systole ventriculaire entraîne la fermeture des valves mitrale et tricuspide. Les bruits cardiaques sont nommés en fonction de leur place dans le cycle cardiaque et se produisent à des points spécifiques de ce dernier. Le bruit cardiaque initial est appelé le premier bruit du cœur B1. Il survient au début de la systole ventriculaire lorsque le volume ventriculaire est maximal. Le premier bruit B1 correspond à un point qui apparaît précocement dans l'élévation de la courbe de pression ventriculaire, lorsque celle-ci devient supérieure à la pression auriculaire et que les valves mitrale et tricuspide se ferment. Cela correspond au complexe QRS de l'ECG (électrocardiogramme). Sur un enregistrement graphique des bruits cardiaques, appelé phonocardiogramme, c'est le premier des composants enregistrés. Le deuxième bruit du cœur B2 se produit à la fin de la systole ventriculaire, au moment de l'onde dicrote sur la courbe de pression ventriculaire, lorsque les valves pulmonaire et aortique se ferment. C'est le deuxième des composants enregistrés sur un phonocardiogramme. La période comprise entre B1 et B2 représente la systole ventriculaire.

Le spectre acoustique cardiaque s'étend typiquement entre 0 et 1300 Hz. Néanmoins, la majorité de la puissance acoustique émise par le cœur se situe en-dessous de 70 Hz.

On rappelle en outre que la hauteur de la voix « parlée » diffère selon le sexe et l'âge, mais se situe typiquement entre 75 Hz et 450 Hz. Cela rend possible la séparation des informations cardiaque et vocale.

Pour la mesure du rythme cardiaque, le dispositif 200 est placé sur le thorax de l'individu, sensiblement sur la gauche, la couche d'adaptation d'impédance 40 étant placée en contact de ses vêtements (dans cet exemple, deux épaisseurs de vêtements en coton et laine).

La figure 6a présente un spectrogramme A, brut, acquis sur une échelle de fréquences allant de 0 à 1300 Hz, par le capteur de vibration 100 conforme à l'invention (fréquence d'acquisition 128 kHz). Le spectrogramme Ref correspond à l'acquisition (en parallèle de la mesure faite par le capteur de vibration 100 du rythme cardiaque) par un microphone classique, du signal relatif à l'environnement sonore : l'individu pour lequel la mesure du signal vital est effectuée parle avec d'autres personnes, l'environnement sonore est donc bruyant, comme cela ressort du spectrogramme de référence Ref.

Sur la figure 6b, un extrait B de 15s du spectrogramme A est reporté, sur une échelle de fréquence 0 - 300 Hz, après application d'un filtre passe-bas à 300 Hz. On remarque que des pics réguliers (pointés par les flèches blanches sur le spectrogramme B), dans la gamme de fréquences 0 - 70 Hz, sont très clairement identifiables : ils correspondent au rythme cardiaque mesuré par le capteur de vibration 100 selon l'invention. En coupant à 300Hz, la majeure partie de l'information relative aux pulsations cardiaques est conservée mais on élimine beaucoup de fréquences parasites notamment liées à la parole et à tout autre bruit environnant entre 300 et 1300Hz que l'on peut subir notamment dans un véhicule automobile, aérien ou nautique (individuel, collectif, de secours, ou autre), ou dans un lieu bruyant en général.

Les pics pointés sur le spectrogramme B peuvent être visualisés sous forme d'onde : c'est le signal C, présenté sur la figure 6b. Un zoom D sur ce signal C révèle les pics représentatifs du premier bruit B1 et du deuxième bruit B2 correspondant au rythme cardiaque de l'individu.

A partir des signaux C et D, il est possible d'extraire le signal périodique et/ou un paramètre de sortie, représentatif du rythme cardiaque de l'individu.

Comme cela vient d'être illustré et de manière générale, le capteur de vibration 100 et le dispositif 200 non intrusif de mesure d'un signal vital périodique selon la présente invention procurent une information fiable quant au signal vital, quel que soit l'environnement sonore et l'activité de l'individu au moment de la mesure ; ils relaxent en outre les contraintes de mesures, puisque celles-ci ne nécessitent pas un contact direct avec la peau de l'individu.

Bien entendu, l'invention n'est pas limitée aux modes de réalisation et aux exemples décrits, et on peut y apporter des variantes de réalisation sans sortir du cadre de l'invention tel que défini par les revendications.

## Revendications

1. Capteur de vibration (100) pour la mesure d'au moins un signal vital périodique d'un individu, comprenant :
- un empilement de couches (10) s'étendant parallèlement à un plan principal (x,y) et incluant une couche active (11) en matériau piézoélectrique et deux électrodes de contact (12,13) disposées sur au moins une face de la couche active (11), ladite couche active (11) présentant une épaisseur inférieure ou égale à 20 microns et un module d'Young supérieur ou égal à 60 GPa,
- une couche support (30), flexible, destinée à se déformer à chaque pulsation du signal vital pour transmettre une déformation à la couche active (11) de l'empilement de couches (10), ladite couche support (30) s'étendant parallèlement au plan principal (x,y) et incluant un circuit imprimé (31) comportant deux bornes électriques (32,33), la couche support (30) étant autoportée et présentant une épaisseur inférieure ou égale à 500µm et une raideur comprise entre 1150000 N/m et 6900000 N/m,
- une couche de connexion électrique (20), disposée entre l'empilement de couches (10) et la couche support (30), pour connecter chaque électrode de contact (12,13) à une borne électrique (32,33),
le capteur de vibration (100) étant destiné à être placé en contact avec l'individu, du côté de la couche support (30).

2. Capteur de vibration (100) selon la revendication précédente, comprenant une couche d'adaptation d'impédance (40), présentant une impédance acoustique comprise entre 5.10⁵ Pa*s/m et 3.10⁶ Pa*s/m, et disposée sur une face de la couche support (30) opposée à celle en contact avec la couche de connexion électrique (20).

3. Capteur de vibration (100) selon l'une des revendications précédentes, dans lequel le matériau piézoélectrique de la couche active (11) est choisi parmi les céramiques sous forme monocristalline, poly-cristalline ou composite.

4. Capteur de vibration (100) selon l'une des revendications précédentes, dans lequel :
- les électrodes de contact (12,12a,13) présentent une épaisseur cumulée inférieure à deux fois l'épaisseur de la couche active (11) ;
- la couche d'adaptation d'impédance (40) présente une épaisseur supérieure ou égale à 10 microns.

5. Capteur de vibration (100) selon l'une des revendications précédentes, dans lequel la couche de connexion électrique (20) est formée par un interposeur ou par un film conductif anisotrope.

6. Capteur de vibration (100) selon l'une des revendications précédentes, dans lequel la couche support (30) inclut une membrane (35) disposée sur une face du circuit imprimé (31) opposée à celle en contact avec la couche de connexion électrique (20).

7. Capteur de vibration (100) selon l'une des revendications précédentes, dans lequel l'empilement de couches (10) et la couche support (30) présentent respectivement une première superficie et une deuxième superficie, dans le plan principal (x,y), la première superficie étant inférieure ou égale à 30% de la deuxième superficie.

8. Capteur de vibration (100) selon l'une des revendications précédentes, dans lequel la couche support (30) comprend une structure de rigidification (50), solidaire d'une zone périphérique de ladite couche support (30).

9. Capteur de vibration (100) selon l'une des revendications précédentes, dans lequel le circuit imprimé (31) comprend un élément de connexion filaire (31b), pour raccorder le capteur de vibration (100) à un terminal électronique (150).

10. Capteur de vibration (100) selon la revendication 8, dans lequel la structure de rigidification (50) supporte deux prises de contact électrique (82,83), chacune reliée à une borne électrique (32,33), pour raccorder le capteur de vibration (100) à un terminal électronique (150).

11. Capteur de vibration (100) selon l'une des revendications précédentes, comprenant un joint périphérique (60).

12. Capteur de vibration (100) selon l'une des revendications précédentes, comprenant une couche de protection (70) disposée au-dessus et à distance de l'empilement de couches (10), ladite couche de protection (70) étant solidaire de la couche support (30).

13. Dispositif (200), non intrusif, pour la mesure d'au moins un signal vital périodique d'un individu, comprenant :
- au moins un capteur de vibration (100) selon l'une des revendications précédentes, pour mesurer un signal brut représentatif du signal vital périodique, et
- un terminal électronique (150) raccordé audit capteur de vibration (100), pour analyser et interpréter le signal brut et extraire le signal vital périodique ou un paramètre de sortie représentatif dudit signal vital périodique.

14. Dispositif (200) selon la revendication précédente, dans lequel le terminal électronique (150) comprend :
- un étage analogique de conditionnement du signal brut mesuré par le capteur de vibration (100),
- un étage de conversion analogique à digital du signal issu de l'étage de conditionnement,
- un étage de traitement du signal digital, pour la mise en forme du signal digital et le calcul d'un paramètre de sortie représentatif dudit signal vital.

15. Dispositif (200) selon l'une des revendications 13 et 14, dans lequel le terminal électronique (150) comprend un étage de communication avec un système externe.

## Patentansprüche

1. Vibrationssensor (100) zur Messung von mindestens einem periodischen Vitalsignal einer Person, umfassend
- einen Schichtstapel (10), der sich parallel zu einer Hauptebene (x, y) erstreckt und eine aktive Schicht (11) aus einem piezoelektrischen Material und zwei Kontaktelektroden (12, 13) einschließt, die auf mindestens einer Seite der aktiven Schicht (11) angeordnet sind, wobei die aktive Schicht (11) eine Dicke von weniger als oder gleich 20 Mikrometern und einen Elastizitätsmodul von mehr als oder gleich 60 GPa aufweist,
- eine flexible Trägerschicht (30), die dazu bestimmt ist, sich bei jedem Pulsieren des Vitalsignals zu verformen, um eine Verformung auf die aktive Schicht (11) des Schichtstapels (10) zu übertragen, wobei sich die Trägerschicht (30) parallel zur Hauptebene (x, y) erstreckt und eine Leiterplatte (31) mit zwei elektrischen Anschlüssen (32, 33) einschließt, wobei die Trägerschicht (30) selbsttragend ist und eine Dicke von weniger als oder gleich 500 µm und eine Steifigkeit zwischen 1150000 N/m und 6900000 N/m aufweist,
- eine elektrische Verbindungsschicht (20), die zwischen dem Schichtstapel (10) und der Trägerschicht (30) angeordnet ist, um jede Kontaktelektrode (12, 13) mit einem elektrischen Anschluss (32, 33) zu verbinden, wobei der Vibrationssensor (100) dazu bestimmt ist, in Kontakt mit der Person auf der Seite der Trägerschicht (30) platziert zu werden.

2. Vibrationssensor (100) nach dem vorstehenden Anspruch, umfassend eine Impedanzanpassungsschicht (40), die eine akustische Impedanz zwischen 5·10⁵ Pa*s/m und 3·10⁶ Pa*s/m aufweist und auf einer Seite der Trägerschicht (30) angeordnet ist, die derjenigen gegenüberliegt, die in Kontakt mit der elektrischen Verbindungsschicht (20) steht.

3. Vibrationssensor (100) nach einem der vorstehenden Ansprüche, wobei das piezoelektrische Material der aktiven Schicht (11) aus Keramiken in einkristalliner, polykristalliner oder Verbundform ausgewählt ist.

4. Vibrationssensor (100) nach einem der vorstehenden Ansprüche, wobei
- die Kontaktelektroden (12, 12a, 13) eine Gesamtdicke aufweisen, die weniger als das Doppelte der Dicke der aktiven Schicht (11) beträgt;
- die Impedanzanpassungsschicht (40) eine Dicke von mehr als oder gleich 10 Mikrometern aufweist.

5. Vibrationssensor (100) nach einem der vorstehenden Ansprüche, wobei die elektrische Verbindungsschicht (20) durch einen Interposer oder durch einen anisotropen leitfähigen Film gebildet ist.

6. Vibrationssensor (100) nach einem der vorstehenden Ansprüche, wobei die Trägerschicht (30) eine Membran (35) einschließt, die auf einer Seite der Leiterplatte (31) angeordnet ist, die derjenigen gegenüberliegt, die in Kontakt mit der elektrischen Verbindungsschicht (20) steht.

7. Vibrationssensor (100) nach einem der vorstehenden Ansprüche, wobei der Schichtstapel (10) und die Trägerschicht (30) eine erste Fläche bzw. eine zweite Fläche in der Hauptebene (x, y) aufweisen, wobei die erste Fläche kleiner oder gleich 30 % der zweiten Fläche ist.

8. Vibrationssensor (100) nach einem der vorstehenden Ansprüche, wobei die Trägerschicht (30) eine Versteifungsstruktur (50) umfasst, die fest mit einem Randbereich der Trägerschicht (30) verbunden ist.

9. Vibrationssensor (100) nach einem der vorstehenden Ansprüche, wobei die Leiterplatte (31) ein Drahtverbindungselement (31b) zum Verbinden des Schwingungssensors (100) mit einem elektronischen Endgerät (150) umfasst.

10. Vibrationssensor (100) nach Anspruch 8, wobei die Versteifungsstruktur (50) zwei elektrische Kontakte (82, 83) trägt, die jeweils mit einem elektrischen Anschluss (32, 33) verbunden sind, um den Vibrationssensor (100) mit einem elektronischen Endgerät (150) zu verbinden.

11. Vibrationssensor (100) nach einem der vorstehenden Ansprüche, umfassend eine Umfangsdichtung (60).

12. Vibrationssensor (100) nach einem der vorstehenden Ansprüche, umfassend eine Schutzschicht (70), die über und in einem Abstand zu dem Schichtstapel (10) angeordnet ist, wobei die Schutzschicht (70) fest mit der Trägerschicht (30) verbunden ist.

13. Nicht-invasive Vorrichtung (200) zur Messung mindestens eines periodischen Vitalsignals einer Person, umfassend:
- mindestens einen Vibrationssensor (100) nach einem der vorstehenden Ansprüche zum Messen eines Rohsignals, das für das periodische Vitalsignal repräsentativ ist, und
- ein mit dem Vibrationssensor (100) verbundenes elektronisches Endgerät (150) zum Analysieren und Interpretieren des Rohsignals und zum Extrahieren des periodischen Vitalsignals oder eines für das periodische Vitalsignal repräsentativen Ausgangsparameters.

14. Vorrichtung (200) nach dem vorstehenden Anspruch, wobei das elektronische Endgerät (150) Folgendes umfasst:
- eine analoge Stufe zur Aufbereitung des vom Vibrationssensor (100) gemessenen Rohsignals,
- eine Stufe zur Analog-Digital-Wandlung des aus der Aufbereitungsstufe kommenden Signals,
- eine digitale Signalverarbeitungsstufe zur Formatierung des digitalen Signals und zur Berechnung eines Ausgangsparameters, der für das Vitalsignal repräsentativ ist.

15. Vorrichtung (200) nach einem der Ansprüche 13 und 14, wobei das elektronische Endgerät (150) eine Stufe zur Kommunikation mit einem externen System umfasst.

## Claims

1. Vibration sensor (100) for measuring at least one vital periodic signal from an individual, comprising
- a stack (10) of layers extending parallel to a main plane (x, y) and including an active layer (11) made of piezoelectric material and two contact electrodes (12, 13) arranged on at least one face of the active layer (11), said active layer (11) having a thickness less than or equal to 20 microns and a Young's modulus greater than or equal to 60GPa,
- a flexible support layer (30), intended to be deformed at each pulsing of the vital signal to transmit a deformation to the active layer (11) of the stack (10) of layers, said support layer (30) extending parallel to the main plane (x, y) and including a printed circuit (31) comprising two electric terminals (32, 33), the support layer (30) being self-supported and having a thickness less than or equal to 500µm and a stiffness of between 1,150,000N/m and 6,900,000N/m,
- an electrical connection layer (20), arranged between the stack (10) of layers and the support layer (30), to connect each contact electrode (12, 13) to an electric terminal (32, 33), the vibration sensor (100) being intended to be placed in contact with the individual, on the side of the support layer (30).

2. Vibration sensor (100) according to the preceding claim, comprising an impedance adaptation layer (40), having an acoustic impedance of between 5.10⁵Pa*s/m and 3.10⁶Pa*s/m, and arranged on a face of the support layer (30) opposite that in contact with the electrical connection layer (20).

3. Vibration sensor (100) according to any one of the preceding claims, wherein the piezoelectric material of the active layer (11) is chosen from among ceramics in monocrystalline, polycrystalline or composite form.

4. Vibration sensor (100) according to any one of the preceding claims, wherein:
- the contact electrodes (12, 12a, 13) having a cumulated thickness less than twice the thickness of the active layer (11);
- the impedance adaptation layer (40) has a thickness greater than or equal to 10 microns.

5. Vibration sensor (100) according to any one of the preceding claims, wherein the electrical connection layer (20) is formed by an interposer or by an anisotropic conductive film.

6. Vibration sensor (100) according to any one of the preceding claims, wherein the support layer (30) includes a membrane (35) arranged on a face of the printed circuit (31) opposite that in contact with the electrical connection layer (20).

7. Vibration sensor (100) according to any one of the preceding claims, wherein the stack (10) of layers and the support layer (30) have respectively a first surface area and a second surface area, in the main plane (x, y), the first surface area being less than or equal to 30% of the second surface area.

8. Vibration sensor (100) according to any one of the preceding claims, wherein the support layer (30) comprises a rigidification structure (50), secured to a peripheral zone of said support layer (30).

9. Vibration sensor (100) according to any one of the preceding claims, wherein the printed circuit (31) comprises a wired connection element (31b), to connect the vibration sensor (100) to an electronic terminal (150).

10. Vibration sensor (100) according to claim 8, wherein the rigidification structure (50) supports two electrical contact outlets (82, 83), each connected to an electrical terminal (32, 33), to connect the vibration sensor (100) to an electronic terminal (150).

11. Vibration sensor (100) according to any one of the preceding claims, comprising a peripheral seal (60).

12. Vibration sensor (100) according to any one of the preceding claims, comprising a protective layer (70) arranged above and at a distance from the stack (10) of layers, said protective layer (70) being secured to the support layer (30).

13. Non-intrusive device (200), for measuring at least one vital periodic signal from an individual, comprising:
- at least one vibration sensor (100) according to any one of the preceding claims, for measuring a raw signal representative of the vital periodic signal, and
- an electronic terminal (150) connected to said vibration sensor (100), for analysing and interpreting the raw signal and extracting the vital periodic signal or an output parameter representative of said vital periodic signal.

14. Device (200) according to the preceding claim, wherein the electronic terminal (150) comprises:
- an analogue stage of conditioning the raw signal measured by the vibration sensor (100),
- a stage for converting the signal coming from the conditioning stage from analogue to digital,
- a stage of processing the digital signal, for shaping the digital signal and calculating an output parameter representative of said vital signal.

15. Device (200) according to any one of claims 13 and 14, wherein the electronic terminal (150) comprises a stage of communicating with an external system.
